# FASCICULE DE BREVET EUROPEEN

(11) **EP 2 191 815 B1**
(45) Date de publication et mention de la délivrance du brevet: **14.03.2018**
(21) Numéro de dépôt: 09168846.5
(22) Date de dépôt: 27.08.2009
(51) Int. Cl.: A61K 8/368, A61Q 9/02

(54) **Procédé de rasage utilisant des dérivés de l'acide salicylique**
Rasierverfahren mit Salicylsäure Derivaten
Method of shaving by using salicylic acid derivatives

(30) Priorité: 08.10.2008 FR 0856811
(43) Date de publication de la demande: 02.06.2010
(73) Titulaire: L'Oréal, 75008 Paris (FR)
(72) Inventeur: Aubert, Lionel, 95270, Asnieres Sur Oise (FR)
(74) Mandataire: Miszputen, Laurent

(56) Documents cités:
- EP-A- 0 378 936
- FR-A- 2 581 542
- FR-A- 2 899 470
- US-A1- 2004 161 392

## Description

La présente invention concerne un procédé de rasage de la peau en particulier de la peau du visage des hommes mettant en oeuvre une composition comprenant dans un milieu cosmétiquement acceptable au moins un dérivé de l'acide salicylique de formule (I) que l'on définira plus loin.

La présente invention a également pour objet l'utilisation d'au moins un dérivé de l'acide salicylique de formule (I) que l'on définira plus loin dans une composition cosmétique de rasage, dans le but de faciliter le rasage du poil.

La barbe d'un homme adulte comprend en moyenne de 8000 à 25000 poils. Le rasage doit être quotidien, en particulier le matin, puisque la barbe pousse environ de 0,4 mm en 24 heures. Si l'on analyse les résidus d'un rasage, on observe 50% de poils et 50% de cellules mortes. Le passage de la lame a donc deux actions simultanées : couper le poil et exfolier la surface cutanée.

Or le rasage est traumatisant pour la peau et il existe un besoin pour disposer de compositions cosmétiques capables de préparer la peau au rasage et ainsi de faciliter celui-ci.

La présente invention vise précisément à offrir un procédé de rasage permettant de faire gonfler le poil, de diminuer sa rigidité pour faciliter le passage de la lame, de réduire les forces de flexion du poil pendant le rasage.

La présente invention vise également à offrir un procédé de rasage permettant une exfoliation chimique de la surface cutanée qui va faciliter le rasage du poil puisque la peau sera débarrassée des cellules mortes encombrant le poil et le rendant plus difficile à couper. Le poil étant ainsi mis à nu et la surface de la peau rendue plus lisse, la lame du rasoir rencontrera moins de résistance et le risque de microcoupures sera diminué. Un autre avantage d'un procédé de rasage permettant une exfoliation chimique de la surface cutanée est de pouvoir diminuer les poils incarnés. En enlevant les cellules mortes en surface, on libère les pores cutanées en douceur, favorisant la pousse du poil. Les poils incarnés créent un inconfort important pour le rasage surtout pour les barbes très frisées qui sont plus difficiles à rendre nettes.

On sait que certains dérivés de l'acide salicylique dans le brevet FR2581542 sont connus pour leurs propriétés kératolytiques et/ou comédolytiques notamment dans le traitement de l'acné. On sait également dans la demande WO2004/073745 que ces mêmes composés salicyliques permettent également de renforcer l'activité d'agents actifs cosmétiques ou dermatologiques.

On sait dans le brevet US 6,156299 que l'acide acétylsalicylique associé à un mélange spécifique comprenant du propylène glycol, de la glycérine, de l'isopropanol et éventuellement de l'éthanol et/ou de l'eau ont été utilisés dans un traitement de la peau hors rasage pour prévenir ou traiter les poils incarnés.

La demanderesse a découvert de manière surprenante qu'en utilisant les dérivés de l'acide salicylique particuliers de formule (I) dans une composition de rasage de la peau, on obtenait d'une part un ramollissement du poil permettant de faciliter le rasage lors du passage de la lame du rasoir. D'autre part, la demanderesse a constaté que ces mêmes composés salicyliques introduits dans une composition de rasage permettaient d'obtenir une exfoliation chimique de la surface cutanée et donc une facilité du rasage ainsi qu'une diminution ou une suppression des poils incarnés. Cette découverte est la base de la présente invention.

La présente invention concerne un procédé de rasage de la peau en particulier de la peau du visage des hommes comprenant au moins les étapes suivantes :
a) on applique sur la zone de peau à raser une composition comprenant dans un milieu cosmétiquement acceptable au moins un dérivé de l'acide salicylique de formule (I) que l'on définira plus loin
b) on rase les poils au moyen d'un rasoir

De manière préférentielle, on masse la zone de peau ainsi traitée pendant un temps allant de 20 secondes à 3 minutes puis on rase les poils et on rince à l'eau après l'étape du rasage.

On entend par « milieu cosmétiquement acceptable » on entend par compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

La présente invention a également pour objet l'utilisation d'au moins un dérivé de l'acide salicylique de formule (I) que l'on définira plus loin dans une composition cosmétique de rasage, dans le but de faciliter le rasage du poil.

La présente invention a également pour objet l'utilisation d'au moins un dérivé de l'acide salicylique de formule (I) que l'on définira plus loin dans une composition cosmétique de rasage, dans le but de diminuer voire d'éliminer les poils incarnés de la peau lors du rasage.

Les dérivés de l'acide salicyliques de l'invention répondent à la formule (I) suivante : 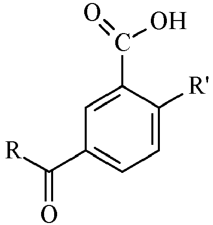 dans laquelle :
R représente une chaîne aliphatique saturée, linéaire, ramifié ou cyclique ayant de 3 à 11 atomes de carbone ; une chaîne aliphatique insaturée ayant de 3 à 17 atomes de carbone portent une ou plusieurs doubles liaisons conjuguées ou non ; un noyau aromatique lié au radical carbonyle directement ou par l'intermédiaire de chaînes aliphatiques saturées ou insaturées ayant de 2 à 7 atomes de carbone ; lesdits groupements R pouvant être substitués par un ou plusieurs susbstituants identiques ou différents choisis parmi les atomes d'halogène, le groupement trifluorométhyle, un groupement hydroxyle sous forme libre ou estérifiée par un acide ayant de 1 à 6 atomes de carbone ou un bien par une fonction carboxyle, libre ou estérifiée par un alcool en C₁-C₆ ;
R' représente un groupe hydroxyle ou une function ester de formule (II) suivante : 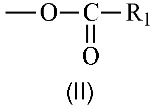 où R₁ représente une chaîne aliphatique de 1 à 18 atomes de carbone linéaire ou ramifiée, saturée ou insaturée et/ou l'un de ses sels.

Parmi les composés de formule (I) préférés, on peut citer ceux dans lesquels R représente une chaîne dérivée de l'acide linoléique, linolénique ou oléique.

Un autre groupe de composés particulièrement intéressants sont ceux pour lesquels R désigne un alkyle linéaire en C₃-C₁₁ et portant une fonction carboxylique libre estérifiée ou salifiée et R' désigne hydroxyle.

Parmi les composés de formule (I) particulièrement préférés, on utilisera ceux dans lesquels R' désigne OH et R un groupe alkyle linéaire en C₃-C₁₁ notamment choisis parmi l'acide 5-n-octanoyl salicylique (acide capryloyl salicylique), l'acide 5-n-décanoyl salicylique, l'acide 5-n-dodécanoyl salicylique, l'acide 5-n-heptyloxy salicylique ou leurs sels.

Parmi les sels des composés de formule (I), on peut citer ceux obtenus avec une base minérale comme les hydroxydes de métal alcalins, d'ammonium : hydroxyde de sodium, potassium ou ammoniaque ; ceux obtenus avec une base organique comme les alcanolamines

On utilisera encore plus particulièrement l'acide 5-n-octanoyl salicylique (ou acide capryloyl salicylique) de formule suivante 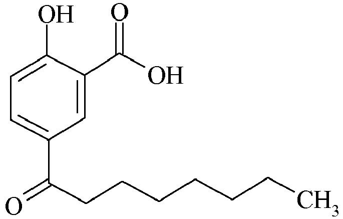 tel que le produit commercial MEXORYL SAB fabriqué par CHIMEX.

Les dérivés de l'acide salicyliques de l'invention de formule (I) sont de préférence présents dans les compositions de rasage à des teneurs allant de 0,05 à 10 %, en particulier de 0,1 à 2 % et notamment de 0,1 % à 1 % en poids par rapport au poids total de la composition.

Les dérivés de l'acide salicylique sont connus en soi. Ils ont été décrits dans le brevet FR2581542 ainsi que leurs procédés de synthèse.

Les compositions de rasage selon l'invention peuvent être formulées sous forme de simples solutions aqueuses qui peuvent être appliquées sur le visage juste avant le rasage. Elles contiennent en général d'autres ingrédients cosmétiques ou dermatologiques choisis par exemple parmi les agents de mouillage de la barbe, les agents conditionneurs de la peau tels que les vitamines A, C, E, l'aloes, l'allantoïne, le panthénol, les hydroxyacides, les phospholipides, les triglycérides, les huiles végétales, les acides aminés), les agents de nettoyage, les agents moussants, les émollients, les hydratants (glycérine, sorbitol, propylène glycol), les tensioactifs (par exemple les savons, les tensioactifs non-ioniques, anioniques ou amphotères), les agents épaississants ou gélifiants, les agents propulseurs, les agents auto-moussants, les parfums, les colorants, les antioxydants, les conservateurs.

Selon une forme particulière de l'invention, les compositions de rasage peuvent se présenter sous forme de lotion, de crème, de mousse ou de gel. De telles compositions comprennent de préférence au moins de 60 à 95% d'eau, plus préférentiellement de 70 à 90% d'eau par rapport au poids total de la composition et de 3 à 25% en poids d'au moins un tensioactif choisi parmi les tensioactifs anioniques, non-ioniques, amphotères ou zwitterioniques ou leurs mélanges et plus préférentiellement de 5 à 20% en poids.

Parmi les tensioactif anioniques utilisables pour l'invention, on peut citer les savons qui sont par exemple les sels de sodium, potassium ou d'alcanolamine (triéthanolamine) d'acides gras en C₁₀-C₂₀ et de préférence en C₁₂-C₁₈. Parmi les savons, on peut mentionner les acides laurique, oléique, d'huile de coco, myristique, palmitique, stéarique ou leurs mélanges.

Parmi les tensioactif anioniques utilisables pour l'invention, on peut citer les sels de sodium, potassium ou d'alcanolamine (triéthanolamine) de N-acylsarcosine comprenant une chaîne grasse dans laquelle le groupe acyle est en C₁₀-C₂₀ et de préférence en C₁₂-C₂₀ comme par exemple les sels de stearoylsarcosine, myristoyl sarcosine, palmitoyl sarcosine, oleoyl sarcosine, lauroyl sarcosine, cocoyl sarcosine et leurs mélanges. Encore plus préférentiellement, elles sont choisies parmi stéaroyl sarcosine, myristoyl sarcosine et leurs mélanges. La ou les sarcosines sont présents à des teneurs allant de 2% à 15% en poids et de préférence allant de 4 à 10% par rapport au poids total de la composition. Les dérivés de N-acylsarcosine peuvent être utilisées sous forme pré-neutralisée ou sous forme acide libre que l'on neutralise par une base comme la soude, la potasse ou l'alcanolamine. Une quantité suffisante de base doit être utilisée pour neutraliser la sarcosine dans la phase aqueuse et produire un pH de 4 à 8.5 et plus préférentiellement de 5 à 7. Pour atteindre cette plage de pH, la sarcosine est de préférence neutralisée de 60 à 80%. On utilisera de préférence la sarcosine en léger excès molaire par rapport à la base. La base est de préférence présente à un taux variant de 1 à 6% par rapport au poids total de la composition.

Parmi les tensioactif anioniques utilisables pour l'invention, on peut également citer les carboxylates, les alkyl sulfates oxyéthylénés ou non, les sulfonates, les alkyl sulfoacétates, les phosphates, les polypeptides, les dérivés anioniques d'alkyl polyglucoside, et leurs mélanges.

Comme carboxylates, on peut citer par exemple :
- les amido éthercarboxylates (AEC), comme le lauryl amido ether carboxylate de sodium (3 OE) commercialisé sous la dénomination AKYPO FOAM 30® par la société Kao Chemicals ;
- les sels d'acides carboxyliques polyoxyéthylénés, comme le lauryl éther carboxylate de sodium (C12-14-16 65/25/10) oxyéthyléné (6 OE) commercialisé sous la dénomination AKYPO SOFT 45 NV® par la société Kao Chemicals ; les acides gras d'huile d'olive polyoxyéthylénés et de carboxyméthyle, produit commercialisé sous la dénomination OLIVEM 400® par la société Biologia E Tecnologia ; le tri-decyl éther carboxylate de sodium oxyéthyléné (6 OE) commercialisé sous la dénomination NIKKOL ECTD-6NEX ® par la société Nikkol ;
- les acétates tels que le 2-(2-hydroxy alkyloxy)acétate de sodium commercialisé sous la dénomination BEAULIGHT SHAA par la société Sanyo ;
- les alaninates comme le N-lauroyl-N-methylamidopropionate de sodium, commercialisé sous la dénomination SODIUM NIKKOL ALANINATE LN 30® par la société Nikkol ou sous la dénomination ALANONE ALE® par la société Kawaken, et le N-lauroyl N-methylalanine triéthanolamine, commercialisé sous la dénomination ALANONE ALTA® par la société Kawaken ; (3) les acylglutamates comme le mono-cocoylglutamate de triéthanolamine commercialisé sous la dénomination ACYLGLUTAMATE CT-12® par la société Ajinomoto, et le lauroyl-glutamate de triéthanolamine commercialisé sous la dénomination ACYLGLUTAMATE LT-12® par la société Ajinomoto ; (4) les aspartates comme le mélange de N-lauroylaspartate de triéthanolamine et de N-myristoylaspartate de triéthanolamine, commercialisé sous la dénomination ASPARACK® par la société Mitsubishi ; (5) les glycinates comme le N-cocoyl glycinate de sodium commercialisé sous les dénominations AMILITE GCS-12® et AMILITE GCK 12 par la société Ajinomoto ;
- les citrates tels que le mono-ester citrique d'alcools de coco oxyéthylénés (9 OA) commercialisé sous la dénomination WITCONOL EC 1129 par la société Goldschmidt ;
- les galacturonates comme le Dodecyl D- galactoside uronate de sodium commercialisé par la société SOLIANCE ;

Comme alkyl sulfates oxyéthylénés ou non, on peut citer par exemple le lauryl éther sulfate de sodium (C12-14 70/30) (2,2 OE) commercialisé sous la dénomination SIPON AOS 225® par la société Cognis, le lauryl éther sulfate d'ammonium (C12-14 70/30) (3 OE) commercialisé sous la dénomination SIPON LEA 370® par la société Cognis, l'alkyl (C12-C14) éther (9 OE) sulfate d'ammonium commercialisé sous la dénomination RHODAPEX AB/20® par la société Rhodia Chimie, le mélange de lauryl et oleyl éther sulfate de sodium et magnésium commercialisé sous la dénomination EMPICOL BSD 52 par la société Albright & Wilson.

Comme sulfonates, on peut citer par exemple
(1) les alpha-oléfines sulfonates comme l'alpha-oléfine sulfonate de sodium (C14-16) commercialisé sous la dénomination BIO-TERGE AS-40 pa s dénominations BIO-TERGE AS-40® et dénomination BIO-TERGE AS-40® par la société Stepan, sous les dénominations WITCONATE AOS PROTEGE® et SULFRAMINE AOS PH 12® par la société Witco, l'oléfine sulfonate de sodium secondaire commercialisé sous la dénomination HOSTAPUR SAS 30® par la société Clariant ;
(2) les iséthionates comme le cocoyl-iséthionate de sodium, tel que le produit commercialisé sous la dénomination JORDAPON CI P® par la société Jordan,
(3) les taurates comme le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo.

Comme phosphates, on peut citer par exemple les monoalkylphosphates et les dialkylphosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea.

Comme polypeptides (qui sont des composés obtenus par condensation d'une chaîne grasse sur les aminoacides de céréale et notamment du blé et de l'avoine), on peut citer par exemple le sel de potassium de la lauroyl protéine de blé hydrolysée, commercialisé sous la dénomination AMINOFOAM W OR® par la société Croda ; le sel de triéthanolamine de cocoyl protéine de soja hydrolysée, commercialisé sous la dénomination MAY-TEIN SY® par la société Maybrook ; le sel de sodium des lauroyl amino-acides d'avoine, commercialisé sous la dénomination PROTEOL OAT® par la société Seppic ; l'hydrolysat de collagène greffé sur l'acide gras de coprah, commercialisé sous la dénomination GELIDERM 3000® par la société Deutsche Gelatine ; les protéines de soja acylées par des acides de coprah hydrogénés, commercialisées sous la dénomination PROTEOL VS 22® par la société Seppic.

Comme dérivés anioniques d'alkyl-polyglucosides, on peut citer notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia ; le sel di-sodique d'ester sulfosuccinique de cocoylpolyglucoside (1,4), commercialisé sous la dénomination ESSAI 512 MP® par la société Seppic ; le sel de sodium d'ester citrique de cocoyl polyglucoside (1,4) commercialisé sous la dénomination EUCAROL AGE-EC® par la société Cesalpinia, le Lauryl polyglucoside éther carboxylate de sodium commercialisé sous la dénomination PLANTAPON LGC SORB par la société Cognis.

On utilisera de préférence les sels d'alkyléthersulfates en C₆-C₂₄ comportant de 1 à 30 groupements oxyde d'éthylène , en particulier les sels de métaux alcalins ou alcalino-terreux, d'ammonium, d'amine ou d'aminoalcool et plus particulièrement les sels de sodium et encore plus particulièrement les alkyl (C₁₂-C₁₄)éthersulfates de sodium oxyéthylénés comportant un nombre moyen de groupes oxyde d'éthylène compris entre 1 et 4 et plus particulièrement le Sodium Laureth Sulfate (nom CTFA).
(i)

Les tensioactifs amphotères et zwitterioniques conformes à l'invention peuvent être choisi parmi les alkylbétaïnes, les N-alkylamidobétaïnes et leurs dérivés, les sultaïnes, les alkyl-polyaminocarboxylate (APAC), les alkylamphoacétates et leurs mélanges.

Comme alkylbétaïnes, on peut citer par exemple la cocobétaïne comme le produit commercialisé sous la dénomination DEHYTON AB-30® par la société Cognis ou les produits commerciaux MIRATAINE BB/FLA de RHODIA ou EMPIGEN BB/FL de Huntsman ; la laurylbétaïne comme le produit commercialisé sous la dénomination GENAGEN KB® par la société Clariant ou le produit commercialisé sous la dénomination EMPIGEN BB / LS® par la société Hunstma,; la laurylbétaïne oxyethylénée (10 OE), comme le produit commercialisé sous la dénomination LAURYLETHER (10 OE) BETAINE® par la société Shin Nihon Rica ; la stéarylbétaïne oxyéthylénée (10 OE) comme le produit commercialisé sous la dénomination STEARYLETHER (10 OE) BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou sous la dénomination EMPIGEN BB® par la société Albright & Wilson ; la lauramidopropyl bétaïne commercialisée sous la dénomination REWOTERIC AMB12P® par la société Witco.

Comme sultaines, on peut citer le cocoyl-amidopropylhydroxy-sulfobetaine commercialisé sous la dénomination CROSULTAINE C-50® par la société Croda.

Comme alkyl-polyaminocarboxylates (APAC), on peut citer le cocoylpolyamino-carboxylate de sodium, commercialisé sous la dénomination AMPHOLAK 7 CX/C®,et AMPHOLAK 7 CX® par la société Akzo Nobel ; le stéaryl-polyamidocarboxylate de sodium commercialisé sous la dénomination AMPHOLAK 7 TX/C® par la société Akzo Nobel ; la carboxyméthyloléyl-polypropylamine de sodium, commercialisé sous la dénomination AMPHOLAK X07/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie ; et le N-cocoyl-N-hydroxyéthyl-N-carboxyméthyl-éthylènediamine N-sodique (nom CTFA : sodium cocamphoacetate).

On utilisera plus particulièrement parmi les tensio-actifs amphotères ou zwittérioniques, les alkylbétaines et encore plus préférentiellement la laurylbétaine et plus particulièrement la lauryl bétaine sous forme de solution aqueuse à 30% en mélange avec du chlorure de sodium (nom INCI : Lauryl Betaine (and) Sodium Chloride) tel que le produit commercial EMPIGEN BB/LS de Huntsman.

Les compositions selon l'invention contiennent un ou plusieurs tensioactifs non ioniques. Ce sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools gras dont la chaîne grasse comporte de préférence de 8 à 2 atomes de carbone ; les alcools, les alpha-diols, les alkylphénols , les acides gras, les acides gras polyéthoxylés, polypropoxylés ou polyglycérolés, ayant une chaîne grasse comportant, de préférence, de 8 à 20 atomes de carbone, et où le nombre de groupements oxyde d'éthylène ou oxyde de propylène varie de préférence de 2 à 60 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant de préférence en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de préférence de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)-aminopropylmorpholine ; et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, sous la dénomination PLANTAREN 2000 UP® par la société Cognis, et sous la dénomination ORAMIX NS 10® par la société Seppic ; le caprylyl/capryl glucoside comme le produit commercialisé sous la dénomination ORAMIX CG 110 ® par la Société Seppic ; le laurylglucoside comme les produits commercialisés sous les dénominations PLANTAREN 1200 N® et PLANTACARE 1200® par la société Cognis ; et le coco-glucoside comme le produit commercialisé sous la dénomination PLANTACARE 818/UP® par la société Cognis.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit fabriqué sous la dénomination CHIMEXANE NF® par la société Chimex.

Les tensioactifs non-ioniques préférentiels sont choisis parmi :
- les alcools gras ont la chaîne grasse est en C₈-C₂₀, plus préférentiellement dont la chaîne grasse est en C₁₂-C₁₈ comme par exemple l'alcool myristique, l'alcool laurique, l'alcool stéarylique et l'octyldodécanol ;
- les éthers polyoxyéthylénés d'alcools gras dont la chaîne grasse est en C₈-C₂₀, plus préférentiellement dont la chaîne grasse est en C₁₂-C₁₈ et ayant de 2 à 60, plus préférentiellement de 2 à 30 unités d'oxyde d'éthylène. Parmi ces composés, on peut citer par exemple Oleth-30, Steareth-21, Ceteth-20, Laureth-4, Laureth-23.

Les compositions de l'invention peuvent contenir en plus un agent gélifiant et par exemple contenir au moins un hydrocarbure liquide non volatil. Les termes « volatil » et « liquide » signifient que ces matériaux sont liquides à température ambiante et ont un point d'ébullition au dessus de 200 °C. Parmi ces hydrocarbures liquides, on peut citer les huiles minérales, les liquides aliphatiques ramifiés. Ces liquides ont de préférence de 16 à 48 atomes de carbone, plus préférentiellement de 20 à 40 atomes de carbone et une viscosité cinétique (mesurée selon la norme ASTM D445) de 5 à 100 cst et plus préférentiellement de 10 à 70 cst à 40 °C. Les hydrocarbures liquides non-volatils préférentiels sont choisis parmi les huiles minérales ayant une viscosité cinétique de 10 à 70 cst, les polyisobutènes hydrogénés de poids moléculaire de 320 à 420 et leurs mélanges. Le ou les hydrocarbures liquides non volatils sont de préférence présents à des concentrations inférieures ou égales à 10% et de préférence inférieures ou égales à 7% en poids par rapport au poids total de la composition.

Les compositions de l'invention peuvent également contenir un agent gélifiant hydrosoluble ou un agent épaississant pour améliorer la consistance et la stabilité du gel ou pour ajuster la viscosité.

Parmi ces agents gelifiants auxiliaires, on peut citer les polymères hydroxyalkylcelluloses comme hydroxyethylcellulose ou hydroxypropylcellulose (produits vendus respectivement sous la dénomination commerciale NATROSOL ou KLUCEL) ; les copolymères d'acide acrylique et de polyallylsucrose (produits vendus sous la dénomination commerciale CARBOPOL) ; la carboxymethylcellulose et la cellulose methyl ether (produits vendus sous la dénomination commerciale METHOCEL) ; les gommes naturelles ou synthétiques, les amidons. Les agents gélifiants ou épaississants sont de préférence présents à des concentrations allant de 0,01 à 5% en poids, plus préférentiellement de 0,05 à 2% en poids et encore plus préférentiellement de 0,01 à 2% en poids par rapport au poids total de la composition.

Les compositions selon l'invention peuvent contenir en plus une variété d'ingrédients cosmétiques classiques pour améliorer les qualités esthétiques et les performances de ces compositions.

Les compositions selon l'invention peuvent également contenir en plus un polymère cationique conditionneur pour améliorer la lubricité et le toucher de la peau après rasage. On peut citer par exemple les sels d'ammonium quaternaires de l'hydroxyyethylcellulose comme Polyquaternium-10, Polyquaternium-24.

On peut également citer les polymères cationiques suivants :
Polyquaternium 5 tel que le produit MERQUAT 5 commercialisé par la société CALGON ;
Polyquaternium 6 tel que le produit SALCARE SC 30 commercialisé par la société CIBA, et le produit MERQUAT 100 commercialisé par la société CALGON ;
Polyquaternium 7 tel que les produits MERQUAT S, MERQUAT 2200 et MERQUAT 550 commercialisés par la société CALGON, et le produit SALCARE SC 10 commercialisé par la société CIBA ;
Polyquaternium 11 tel que les produits GAFQUAT 755, GAFQUAT 755N et GAFQUAT 734 commercialisés par la société ISP ;
Polyquaternium 15 tel que le produit ROHAGIT KF 720 F commercialisé par la société ROHM;
Polyquaternium 16 tel que les produits LUVIQUAT FC905, LUVIQUAT FC370, LUVIQUAT HM552 et LUVIQUAT FC550 commercialisés par la société BASF ;
Polyquaternium 22 tel que le produit MERQUAT 280 commercialisé par la société CALGON ;
Polyquaternium 28 tel que le produit STYLEZE CC10 commercialisé par la société ISP ;
Polyquaternium 39 tel que le produit MERQUAT PLUS 3330 commercialisé par la société CALGON ;
Polyquaternium 44 tel que le produit LUVIQUAT CARE commercialisé par la société BASF;
Polyquaternium 46 tel que le produit LUVIQUAT HOLD commercialisé par la société BASF;
Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

Le ou les polymères cationiques conditionneurs sont présents de préférence à des concentrations allant de 0,05 à 2% en poids, plus préférentiellement allant de 0,1 à 1% en poids par rapport au poids total de la composition.

D'autres additifs peuvent également être utilisés dans les compositions de l'invention comme
- les humectants comme le sorbitol ;
- les émollients tels que les esters gras comme l'isopropyl myristate, le decyl oleate, le 2-ethyhexyl palmitate, le PEG-7 Glyceryl Cocoate et le Glyceryl Linoleate ; les éthers gras propoxylés comme PPG-10 Cetyl Ether et PPG-11 Stearyl Ether; les di- ou triglycérides comme la lécithine, le mélange de triglycérides caprique/caprylique, le PEG-10 Soy Sterol, les huiles végétales.
- les agents rafraichissants et les agents apaisants comme le menthol, l'aloe, l'allantoine, la lanoline, le bisabolol, l'acide hyaluronique ;
- les lubrifiants comme les polyethyleneglycols (ie PEG-14M, PEG-23M), les tensioactifs fluorés, les silicones (ie : dimethicone, dimethiconol, dimethicone copolyol, stearyl dimethicone, cetyl dimethicone copolyol, cyclomethicone, etc...)
- les vitamines incluant les précurseurs et les dérivés comme le panthenol, le tocopheryl acetate, le niacinamide, le retinyl palmitate, le vitamine A palmitate ;
- les colorants ;
- les parfums ;
- les antioxydants ;
- les antibactériens et/ou les antifongiques ;
- les conservateurs (ie : methylchloroisothiazolinone, methylisothiazolinone, DMDM hydantoine, iodopropinyl butylcarbamate).
- les charges.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyl-lysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Selon une forme particulière de l'invention, les compositions de rasage peuvent se présenter sous forme de mousse à raser. Les mousses à raser sont en général sous la forme d'émulsion huile dans eau dans laquelle la phase aqueuse contient en général un tensioactif anionique hydrosoluble. Le produit est généralement conditionné dans un récipient aérosol monobloc dans lequel l'agent propulseur est en mélange avec le jus ; la mousse se forme à la sortie du dispositif aérosol.

L'agent propulseur est choisi de préférence parmi les hydrocarbures volatils et les hydrocarbures volatils halogénés. Le point d'ébullition de l'agent propuleur varie de préférence de -20 à 40°C. Les agents propulseurs utilisables selon l'invention sont choisis parmi les hydrocarbures aliphatiques en C₄-C₆ tels que n-pentane, isopentane, néopentane, n-butane, isobutane et leurs mélanges. Plus préférentiellement, on utilisera un mélange isopentane/butane/propane. L'agent propulseur est de préférence présent à des concentrations allant de 1 à 10% en poids et plus préférentiellement de 2 à 6% en poids par rapport au poids total de la composition.

Selon une forme particulière de l'invention, les compositions de rasage peuvent se présenter sous forme de gel auto-moussant. De telles formulations se présentent sous la forme d'une émulsion huile-dans-eau dans laquelle l'agent auto-moussant, généralement un hydrocarbure aliphatique volatil (ie : de faible point d'ébullition) est solubilisé dans la phase huileuse et la phase aqueuse comprend en général un tensioactif anionique hydrosoluble. Le produit est généralement conditionné dans un récipient aérosol avec une séparation telle qu'un piston ou une poche souple pour séparer l'agent auto-moussant de l'agent propulseur nécessaire pour l'expulsion du produit. Le dispositif peut être également un tube souple ; un flacon pompe ou un flacon à paroi déformable. Le produit est appliqué sous forme de gel transparent, translucide ou opaque qui est substantiellement sans mousse jusqu'à l'étalement sur la peau, moment à partir duquel il se produit une mousse par évaporation de l'agent moussant hydrocarbure volatil.

L'agent auto-moussant est choisi de préférence parmi les hydrocarbures volatils et les hydrocarbures volatils halogénés ayant un point d'ébullition suffisamment bas pour permettre à ces derniers de s'évaporer et de mousser le gel à l'application sur la peau et un point d'ébullition suffisamment haut pour éviter de produire une mousse prématurément. Le point d'ébullition de l'agent auto-moussant varie de préférence de -20 à 40°C. L'agent auto-moussant est choisi de préférence de telle sorte à former une pression de vapeur à 20°C de 3 à 20 psig et de préférence de 5 à 15 psig. Les agents auto-moussants utilisables selon l'invention sont choisis parmi les hydrocarbures aliphatiques en C₄-C₆ tels que n-pentane, isopentane, néopentane, n-butane, isobutane et leurs mélanges. Plus préférentiellement, on utilisera un mélange isopentane/isobutane dans un rapport en poids allant de 1/1 à 3/1. L'agent auto-moussant est de préférence présent à des concentrations allant de 1 à 8% en poids et plus préférentiellement de 2 à 5% en poids par rapport au poids total de la composition.

L'invention concerne également un kit de rasage caractérisé par le fait qu'il comprend
a) au moins une composition de rasage telle que définie ci-dessus et
(b) au moins un rasoir notamment jetable et éventuellement
(c) un moyen d'étalement d'une composition de rasage.

Selon une forme particulière de l'invention, le kit peut contenir en plus une composition après-rasage pour apaiser le feu du rasage.

Les exemples qui suivent servent à illustrer l'invention. Les quantités indiquées sont en % en poids par rapport au poids total de la composition et les noms des composés en noms chimiques ou noms INCI selon les cas.

### EXEMPLES 1 et 2 : Jus Mousse à raser

| **Ingrédients** | **Ex1(*)** | **Ex2** |
|---|---|---|
| Acide Stéarique | 5.16 | 5.16 |
| Acide Palmitique | 4.30 | 4.30 |
| Acide Myristique | 0.29 | 0.29 |
| Glycérine | 3 | 3 |
| **Acide capryloyl salicylique** | - | 0,3 |
| HydroxyPropylCellulose (KLUCEL MF de AQUALON) | 0,10 | 0,10 |
| Triéthanolamine | 5 | 5 |
| Steareth-2 | 1.50 | 1.50 |
| Steareth-20 | 1.50 | 1.50 |
| Caprylic/Capric Triglyceride | 2 | 2 |
| Parfum | 1 | 1 |
| Conservateurs | qs | qs |
| Eau desionisée | qsp 100 | qsp 100 |
| **% Diminution de la rigidité du poil** | 13,6% | 25% |

| | | |
|---|---|---|
| (*) hors invention | | |

On effectue un test comparatif entre ces deux jus de mousses à raser ayant pour objectif de montrer leur influence sur le gonflement du poil et son ramollissement lorsque celui-ci est immergé dans une composition de rasage après avoir été préalablement immergé dans l'eau. On mesure la force de flexion de la fibre ainsi immergée.

Des échantillons de poil sont insérés dans des plaquettes éprouvettes en plastique du type « fibre tip » comportant une extrémité libre. La longueur de l'échantillon de poil libre subissant le test est de 5 mm. Le poil est orienté dans le sens racine-pointe.

Les échantillons seront immergés pendant 10 minutes dans de l'eau puis 10 minutes dans la composition de rasage 1 ou 2.

On mesure après immersion, la force de flexion du poil traité ou non traité par le produit de rasage au moyen d'un appareil du type Diastron FBS 900 qui enregistre la force nécessaire pour courber la fibre. Cet appareil est équipé d'un cylindre rotatif. Il permet des mesures sur différents axe du poil afin de prendre en compte son ellipticité. A partir de la force mesurée puis normalisée par les dimensions de l'échantillon, on calcule la contrainte er on détermine le module de flexion.

Les paramètres utilisés avec cet appareil sont
Force minimale de détection : 2mg
Vitesse de déplacement : 0,05 mm/s
Déplacement vertical maximum : 0,4 mm
Angle : 0°
Distance Cylindre/Eprouvette : 2 mm
Taux d'acquisition : 10 Hz

On calcule ensuite le taux de diminution de la rigidité du poil traité par la composition de rasage par rapport à celle mesurée avec le poil non traité (immergé dans l'eau). On observe que la composition 2 selon l'invention comprenant l'acide capryloyl salicylique permet de ramollir d'avantage le poil et donc d'améliorer le rasage par rapport à la composition 1 ne comprenant pas de dérivé salicylique.

## Revendications

1. Procédé de rasage de la peau en particulier de la peau du visage des hommes comprenant au moins les étapes suivantes :
a) on applique sur la zone de peau à raser une composition comprenant dans un milieu cosmétiquement acceptable au moins un dérivé de l'acide salicylique de formule (I) suivante : 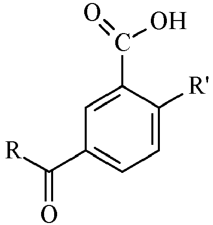 dans laquelle :
R représente une chaîne aliphatique saturée, linéaire, ramifié ou cyclique ayant de 3 à 11 atomes de carbone ; une chaîne aliphatique insaturée ayant de 3 à 17 atomes de carbone portent une ou plusieurs doubles liaisons conjuguées ou non ; un noyau aromatique lié au radical carbonyle directement ou par l'intermédiaire de chaînes aliphatiques saturées ou insaturées ayant de 2 à 7 atomes de carbone ; lesdits groupements R pouvant être substitués par un ou plusieurs susbstituants identiques ou différents choisis parmi les atomes d'halogène, le groupement trifluorométhyle, un groupement hydroxyle sous forme libre ou estérifiée par un acide ayant de 1 à 6 atomes de carbone ou un bien par une fonction carboxyle, libre ou estérifiée par un alcool en C₁-C₆ ;
R' représente un groupe hydroxyle ou une function ester de formule (II) suivante : 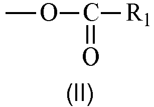 où R₁ représente une chaîne aliphatique de 1 à 18 atomes de carbone linéaire ou ramifiée, saturée ou insaturée et/ou l'un de ses sels.
b) on rase les poils au moyen d'un rasoir.

2. Procédé selon la revendication 1, dans lequel on massera la zone de peau traitée pendant un temps allant de 20 secondes à 3 minutes puis on rase les poils et on rince à l'eau.

3. Procédé selon la revendication 1 ou 2, selon lequel les composés de formule (I) sont choisis parmi ceux pour lesquels R représente une chaîne dérivée de l'acide linoléique, linolénique ou oléique.

4. Procédé selon la revendication 1 ou 2, selon lequel les composés de formule (I) sont choisis parmi ceux pour lesquels R désigne un alkyle linéaire en C₃-C₁₁ et portant une fonction carboxylique libre estérifiée ou salifiée et R' désigne hydroxyle.

5. Procédé selon la revendication 1 ou 2, selon lequel les composés de formule (I) sont choisis parmi ceux pour lesquels R' désigne OH et R un groupe alkyle linéaire en C₃-C₁₁.

6. Procédé selon la revendication 5, selon lequel le composé de formule (I) est l'acide 5-n-octanoyl salicylique (ou acide capryloyl salicylique) de formule suivante

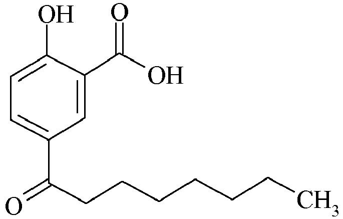

7. Procédé selon l'une quelconque des revendications 1 à 6, où le ou les dérivés de l'acide salicyliques de formule (I) sont présents dans la composition de rasage à des teneurs allant de 0,05 à 10 %, en particulier de 0,1 à 2 % et notamment de 0,1 % à 1 % en poids par rapport au poids total de la composition.

8. Procédé selon l'une quelconque des revendications 1 à 6, où la composition de rasage se présente sous forme de lotion, de crème, de gel, de mousse, de gel auto-moussant.

9. Utilisation d'au moins un dérivé de l'acide salicylique de formule (I) et/ou l'un de ses sels tel que défini dans l'une quelconque des revendications précédentes dans une composition cosmétique de rasage, dans le but de faciliter le rasage du poil.

10. Utilisation d'au moins un dérivé de l'acide salicylique de formule (I) et/ou l'un de ses sels tel que défini dans l'une quelconque des revendications précédentes dans une composition cosmétique de rasage, dans le but de diminuer voire d'éliminer les poils incarnés de la peau lors du rasage.

11. Kit de rasage **caractérisé par le fait qu'**il comprend
a) au moins une composition de rasage telle que définie dans l'une quelconque des revendications précédentes et
(b) au moins un rasoir notamment jetable et éventuellement
(c) un moyen d'étalement d'une composition de rasage.

## Patentansprüche

1. Verfahren zu Rasieren der Haut, insbesondere der Gesichtshaut von Männern, das mindestens die folgenden Schritte umfasst:
a) Aufbringen einer Zusammensetzung, die in einem kosmetisch unbedenklichen Medium mindestens ein Salicylsäurederivat der folgenden Formel (I): 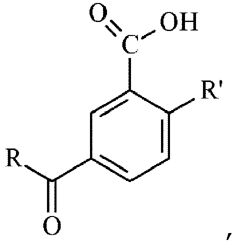 in der:
R für eine lineare, verzweigte oder cyclische, gesättigte aliphatische Kette mit 3 bis 11 Kohlenstoffatomen; eine ungesättigte aliphatische Kette mit 3 bis 17 Kohlenstoffatomen, die eine oder mehrere konjugierte oder nicht konjugierte Doppelbindungen trägt; oder einen aromatischen Kern, der direkt oder über gesättigte oder ungesättigte aliphatische Ketten mit 2 bis 7 Kohlenstoffatomen an den Carbonylrest gebunden ist, steht; wobei die Gruppen R durch einen oder mehrere gleiche oder verschiedene Substituenten, die aus Halogenatomen, der Trifluormethylgruppe, einer Hydroxylgruppe in freier Form oder mit einer Säure mit 1 bis 6 Kohlenstoffatomen veresterter Form ausgewählt sind, oder auch durch eine freie oder mit einem C₁-C₆-Alkohol veresterte Carboxylfunktion substituiert sein können;
R' für eine Hydroxylgruppe oder eine Esterfunktion der folgenden Formel (II) : 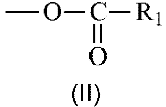 steht, wobei R₁ für eine lineare oder verzweigte, gesättigte oder ungesättigte aliphatische Kette mit 1 bis 18 Kohlenstoffatomen steht, und/oder ein Salz davon umfasst, auf die zu rasierende Hautpartie,
b) Abrasieren der Haare mit einem Rasierer.

2. Verfahren nach Anspruch 1, bei dem man die behandelte Hautpartie über einen Zeitraum im Bereich von 20 Sekunden bis 3 Minuten massiert, dann die Haare abrasiert und mit Wasser spült.

3. Verfahren nach Anspruch 1 oder 2, gemäß dem die Verbindungen der Formel (I) aus denjenigen ausgewählt sind, für die R für eine von Linolsäure, Linolensäure oder Ölsäure abgeleitete Kette steht.

4. Verfahren nach Anspruch 1 oder 2, gemäß dem die Verbindungen der Formel (I) aus denjenigen ausgewählt sind, für die R für ein lineares C₃-C₁₁-Alkyl mit einer freien, veresterten oder versalzten Carboxylfunktion steht und R' für Hydroxyl steht.

5. Verfahren nach Anspruch 1 oder 2, gemäß dem die Verbindungen der Formel (I) aus denjenigen ausgewählt sind, für die R' für OH steht und R für eine lineare C₃-C₁₁-Alkylgruppe steht.

6. Verfahren nach Anspruch 5, gemäß dem es sich bei der Verbindung der Formel (I) um 5-n-Octanoylsalicylsäure (oder Capryloylsalicylsäure) der folgenden Formel 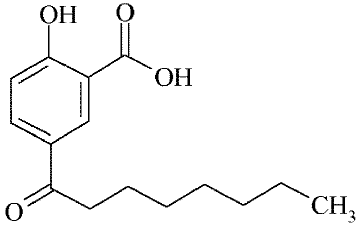 handelt.

7. Verfahren nach einem der Ansprüche 1 bis 6, wobei das Salicylsäurederivat bzw. die Salicylsäurederivate der Formel (I) in der Rasierzusammensetzung in Gehalten im Bereich von 0,05 bis 10 Gew.-%, insbesondere 0,1 bis 2 Gew.-% und speziell 0,1 bis 1 Gew.-%, bezogen auf das Gesamtgewicht der Zusammensetzung, vorliegt bzw. vorliegen.

8. Verfahren nach einem der Ansprüche 1 bis 6, wobei die Rasierzusammensetzung in Form einer Lotion, einer Creme, eines Gels, eines Schaums oder eines selbstschäumenden Gels vorliegt.

9. Verwendung mindestens eines Salicylsäurederivats der Formel (I) und/oder eines Salzes davon gemäß einem der vorhergehenden Ansprüche in einer kosmetischen Rasierzusammensetzung mit dem Ziel der Erleichterung des Abrasierens des Haars.

10. Verwendung mindestens eines Salicylsäurederivats der Formel (I) und/oder eines Salzes davon gemäß einem der vorhergehenden Ansprüche in einer kosmetischen Rasierzusammensetzung mit dem Ziel der Verringerung oder sogar Eliminierung der in die Haut eingewachsenen Haare bei der Rasur.

11. Rasurkit, **dadurch gekennzeichnet, dass** es Folgendes umfasst:
(a) mindestens eine Rasierzusammensetzung gemäß einem der vorhergehenden Ansprüche und
(b) mindestens einen Rasierer, insbesondere einen Einwegrasierer, und gegebenenfalls
(c) ein Mittel zum Verteilen einer Rasierzusammensetzung.

## Claims

1. Method of shaving the skin, in particular the facial skin of men, comprising at least the following steps:
a) a composition comprising, in a cosmetically acceptable medium, at least one salicylic acid derivative of formula (I) below: 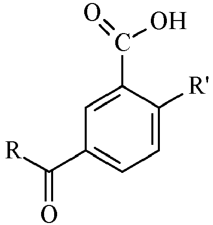 in which:
R represents a linear, branched or cyclic, saturated aliphatic chain containing from 3 to 11 carbon atoms; an unsaturated aliphatic chain containing from 3 to 17 carbon atoms bearing one or more conjugated or nonconjugated double bonds; an aromatic nucleus linked to the carbonyl radical directly or by means of saturated or unsaturated aliphatic chains containing from 2 to 7 carbon atoms; it being possible for said R groups to be substituted with one or more substituents, which may be identical or different, chosen from halogen atoms, a trifluoromethyl group, a hydroxyl group in free form or in a form esterified with an acid containing from 1 to 6 carbon atoms or else with a carboxyl function, which is free or esterified with a C₁-C₆ alcohol;
R' represents a hydroxyl group or an ester function of formula (II) below: 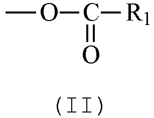 where R₁ represents a saturated or unsaturated, linear or branched aliphatic chain containing from 1 to 18 carbon atoms, and/or a salt thereof, is applied to the area of skin to be shaved;
b) the hairs are shaved by means of a razor.

2. Method according to Claim 1, in which the area of skin treated is massaged for a period of time ranging from 20 seconds to 3 minutes, then the hairs are shaved, followed by rinsing with water.

3. Method according to Claim 1 or 2, according to which the compounds of formula (I) are chosen from those for which R represents a chain derived from linoleic acid, linolenic acid or oleic acid.

4. Method according to Claim 1 or 2, according to which the compounds of formula (I) are chosen from those for which R denotes a C₃-C₁₁ linear alkyl bearing a free, esterified or salified carboxylic function, and R' denotes hydroxyl.

5. Method according to Claim 1 or 2, according to which the compounds of formula (I) are chosen from those for which R' denotes OH and R a C₃-C₁₁ linear alkyl group.

6. Method according to Claim 5, according to which the compound of formula (I) is 5-n-octanoylsalicylic acid (or capryloylsalicylic acid) having the formula below:

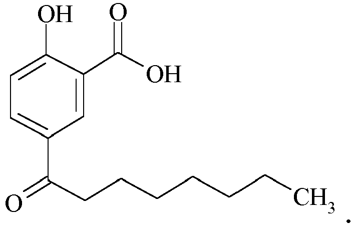

7. Method according to any one of Claims 1 to 6, wherein the salicylic acid derivative(s) of formula (I) is (are) present in the shaving composition at contents ranging from 0.05% to 10%, in particular from 0.1% to 2%, and especially from 0.1% to 1% by weight, relative to the total weight of the composition.

8. Method according to any one of Claims 1 to 6, wherein the shaving composition is in the form of a lotion, a cream, a gel, a foam or a self-foaming gel.

9. Use of at least one salicylic acid derivative of formula (I) and/or a salt thereof as defined in any one of the preceding claims, in a cosmetic shaving composition, with the aim of facilitating the shaving of the hair.

10. Use of at least one salicylic acid derivative of formula (I) and/or a salt thereof as defined in any one of the preceding claims, in a cosmetic shaving composition, with the aim of decreasing or even eliminating the ingrown hairs of the skin during shaving.

11. Shaving kit, **characterized in that** it comprises:
(a) at least one shaving composition as defined in any one of the preceding claims and
(b) at least one razor, in particular a disposable razor, and optionally
(c) a means for spreading a shaving composition.
